# EUROPEAN PATENT APPLICATION

(11) **EP 2 404 545 A2**
(43) Date of publication of application: **11.01.2012**
(21) Application number: 11172522.2
(22) Date of filing: 04.07.2011
(51) Int. Cl.: A61B 3/12

(54) **Fundus photographing apparatus**

(30) Priority: 05.07.2010 JP 2010153420
(71) Applicant: Nidek Co., Ltd., Gamagori-shi, Aichi 443-0038 (JP)
(72) Inventor: Yamamoto, Mitsuo, Gamagori-shi Aichi, 443-0034 (JP); Taki, Seiji, Okazaki-shi Aichi, 444-0201 (JP); Kobayashi Toshihiro, Gamagori-shi Aichi, 443-0002 (JP)
(74) Representative: Zimmermann, Tankred Klaus

(57) **Abstract**

A fundus photographing apparatus for photographing a fundus of an examinee's eye includes: a fundus photographing optical system for obtaining a fundus image, including: an optical scanner that scans the fundus with measurement light including at least part of light emitted from a light source; and a light detector that receives light including reflected light from the fundus; a length information obtaining unit for obtaining length information on an axial direction of the eye; and a controller that adjusts driving information of the fundus photographing optical system in relation to a photographing range based on the length information obtained by the length information obtaining unit and controls the fundus photographing optical system based on the adjusted driving information to obtain a fundus image corresponding to a photographing range.

## Description

### BACKGROUND

### 1. Technical Field

Embodiments described herein relate to a fundus photographing apparatus for photographing a fundus of an examinee's eye.

### 2. Related Art

Fundus tomographic image photographing apparatuses (e.g., Optical Coherence Tomography: OCT) and fundus front image photographing apparatuses (e.g., Scanning Laser Ophthalmoscope: SLO), for example, have been known as apparatuses for obtaining fundus images by scanning the fundus with the measurement light using optical scanning parts. An example of such apparatuses is disclosed in JP-A-2008-29467.

### SUMMARY

A fundus photographing apparatus for photographing a fundus of an examinee's eye includes: a fundus photographing optical system for obtaining a fundus image, including: an optical scanner that scans the fundus with measurement light including at least part of light emitted from a light source; and a light detector (104) that receives light including reflected light from the fundus; a length information obtaining unit (110) for obtaining length information on an axial direction of the eye; and a controller that adjusts driving information of the fundus photographing optical system in relation to a photographing range based on the length information obtained by the length information obtaining unit and controls the fundus photographing optical system based on the adjusted driving information to obtain a fundus image corresponding to a photographing range.

### BRIEF DESCRIPTION OF DRAWINGS

Fig. 1 is a schematic diagram showing an optical system and a control system of a fundus photographing apparatus according to a present embodiment;
Fig. 2 is a schematic diagram explaining a relationship among a scan angle of measurement light, an ocular axial length, and a photographing range;
Fig. 3 is a flowchart showing an exemplary procedure for measuring a fundus image by changing operation of an optical scanner in accordance with the ocular axial length;
Figs. 4A and 4B are schematic explanatory diagrams showing an exemplary calculation technique to determine the scan angle corresponding to an ocular axial length;
Fig. 5 is a diagram showing a front image and a tomographic image provided in accordance with a predetermined photographing range;
Figs. 6A and 6B are examples showing a change of the photographing range in accordance with the ocular axial length; and
Fig. 7 is a diagram showing a specific example of the optical system and the control system of the fundus photographing apparatus according to the present embodiment.

### DESCRIPTION OF EMBODIMENTS

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

In apparatuses of the related art, fundus images are photographed at a certain scanning view angle (scan angle). However, in the case of a different ocular axial length, a photographing range changes despite the same view angle. For example, when the size (length) of a lesion or other areas of the fundus is measured, quantitative evaluation of the size becomes difficult.

A technical problem of the fundus photographing apparatuses is to provide a fundus photographing apparatus capable of performing quantitative evaluation by use of a fundus image.

A fundus photographing apparatus of one embodiment may include the following configuration to solve the problem.

A fundus photographing apparatus for photographing a fundus of an examinee's eye includes: a fundus photographing optical system for obtaining a fundus image, including: an optical scanner that scans the fundus with measurement light including at least part of light emitted from a light source; and a light detector (104) that receives light including reflected light from the fundus; a length information obtaining unit (110) for obtaining length information on an axial direction of the eye; and a controller that adjusts driving information of the fundus photographing optical system in relation to a photographing range based on the length information obtained by the length information obtaining unit and controls the fundus photographing optical system based on the adjusted driving information to obtain a fundus image corresponding to a photographing range.

According to such a configuration, the quantitative evaluation can be performed by use of the fundus image.

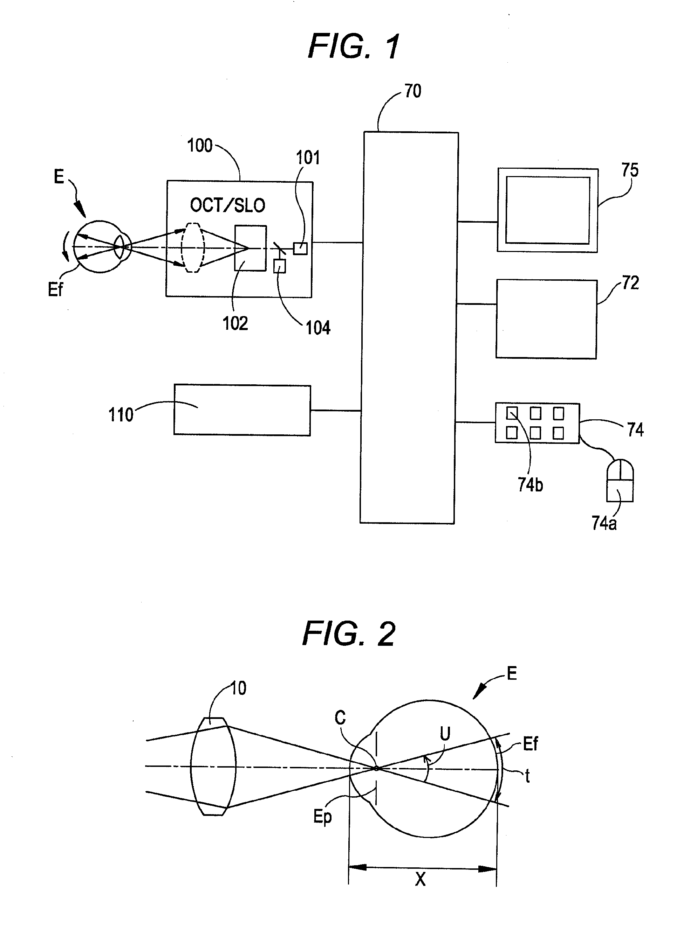

A fundus photographing apparatus according to embodiments is described based on the accompanying drawings. Fig. 1 is a schematic diagram showing an optical system and a control system in the fundus photographing apparatus (present apparatus) according to the present embodiment. In the present embodiment, a description is given with an axial direction of an examinee's eye (eye E) referred to as a Z-direction (direction of optical axis L1), a horizontal direction referred to as an X-direction, and a vertical direction referred to as a Y-direction.

The present apparatus includes a photographing optical system 100, an eye distance measurement apparatus 110, a control part 70, a display monitor 75, a memory 72, and an operating part 74. The photographing optical system 100 obtains an image by scanning the eye E with the measurement light using an optical scanner 102. The eye distance measurement apparatus 110 measures information of an eye length in an axial direction (distance between tissues). The control part 70 obtains the length information from the measurement apparatus 110. The control part 70, based on the obtained length information, corrects scan information (e.g., scan angle) of the measurement light that scans the fundus. The control part 70 controls the optical scanner 102 based on the corrected scan information. The control part 70 obtains a fundus image based on a light receiving signal from a light receiving device 104 (refer to Figs. 2, 3, and 4).

The photographing optical system 100 is provided so as to obtain the fundus image. The photographing optical system 100 includes the optical scanner 102 and the light receiving device 104. The optical scanner 102 scans a fundus Ef with the measurement light including at least part of light emitted from a light source 101. The light receiving device 104 receives light including the reflected light from the fundus Ef.

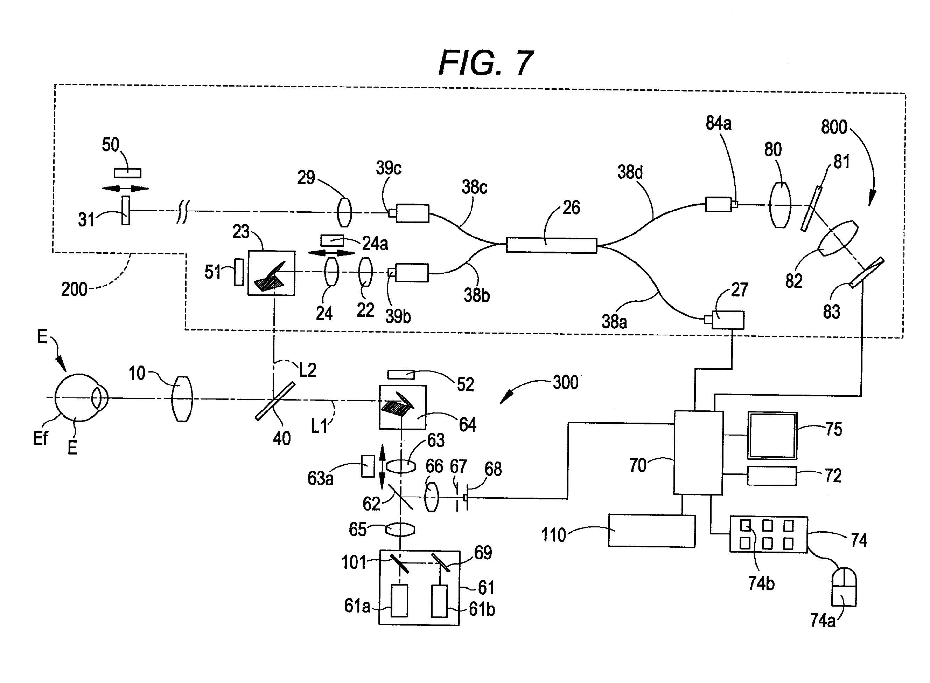

As the photographing optical system 100, for example, at least either an optical system for obtaining a tomographic image of the eye E by optical scanning (refer to an interference optical system 200 of Fig. 7) or an optical system for obtaining a front image of the eye E by optical scanning (refer to an SLO optical system 300 of Fig. 7) can be used. Alternatively, both of these systems may be used as the photographing optical system 100.

The optical scanner (optical scanning part) 102 is arranged in an optical path of the measurement light. The optical scanner 102 changes (deflects) a traveling direction of measurement light flux, so that a scanning position of the measurement light is moved along X- and Y-directions. As the optical scanner 102, a reflection mirror (e.g., galvanometer mirror, polygon mirror, resonant scanner) for changing a reflecting direction of light, an acousto-optic device (Acousto-Optic Modulator: AOM) for changing a traveling direction of light, and the like can be used.

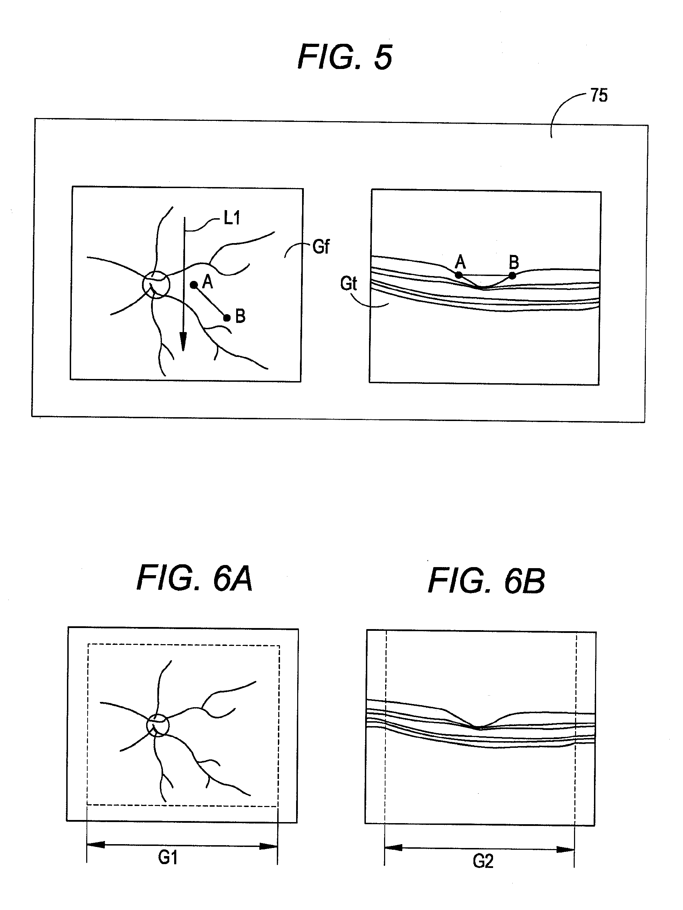

The control part 70 controls driving of the optical scanner 102. The control part 70 performs image processing on the light receiving signal output from the light receiving device 104 to form the fundus image. The obtained fundus image is displayed as a still image or a moving image on the monitor 75 (refer to a front image Gf and a tomographic image Gt of Fig. 5), and is stored in the memory 72.

The measurement apparatus 110 irradiates the eye E with the light or ultrasonic waves to obtain a reflected signal thereof. The measurement apparatus 110 measures a length of the eye E (e.g., ocular axial length, distance from anterior surface of crystalline lens to retinal surface) based on the reflected signal. The measurement apparatus 110 is arranged, for example, in the same housing as the photographing optical system 100. The measurement apparatus 110 may be arranged as another apparatus. In such a case, a measurement result obtained by the measurement apparatus 110 is output to the housing of the photographing optical system 100.

The control part 70 controls the present apparatus as a whole and performs various calculations. The control part 70 is connected with members of the photographing optical system 100, the measurement apparatus 110, the display monitor 75, the memory 72, the operating part 74 for performing a variety of operations, and the like. The control part 70 is connected to the display monitor 75 and controls an image displayed thereon. It is to be noted that a monitor for alignment observation and a monitor for photographed image observation may be individually provided as the display monitor 75, or one shared monitor may be used as the display monitor 75. The operating part 74 includes a measurement position setting part (setting unit, e.g., mouse) 74a and a photographing start switch 74b.

Moreover, the control part 70 obtains at least any of diopter information and corneal shape information of the eye E. The control part 70 can correct the scan information based on the length information and at least one of the diopter information and the corneal shape information obtained. The scan information is used to operate the optical scanner 102.

Fig. 2 is a schematic diagram explaining a relationship among a scan angle of the measurement light, an ocular axial length, and a photographing range. A fundus image (tomographic image or front image) has a photographing range (obtaining range) t that is determined by a scan angle U and an ocular axial length X. The fundus is scanned with light while a pupil center of the eye E is set as a scanning center C. A photographing view angle is determined by the scan angle of the light with respect to the fundus Ef. In the following description, the photographing range t is a range on a surface of the fundus Ef.

Accordingly, the greater the scan angle U, the greater the photographing range t. The photographing range in a vertical direction is determined in accordance with the scan angle in a vertical direction. The photographing range in a horizontal direction is determined in accordance with the scan angle in a horizontal direction. The scan angle with respect to the eye E is determined by a deflection angle of the optical scanner 102.

A position (height) of the measurement light that has reached the fundus varies depending on a length of the ocular axial length X. Herein, the longer the ocular axial length X, the higher the position of the measurement light in the fundus, whereas the shorter the ocular axial length X, the lower the position of the measurement light in the fundus. Therefore, a longer ocular axial length X allows the photographing range t to be greater even if the scan angle is constant. It is to be noted that the photographing range t may be calculated based on a linear distance between a start point and an end point of scanning.

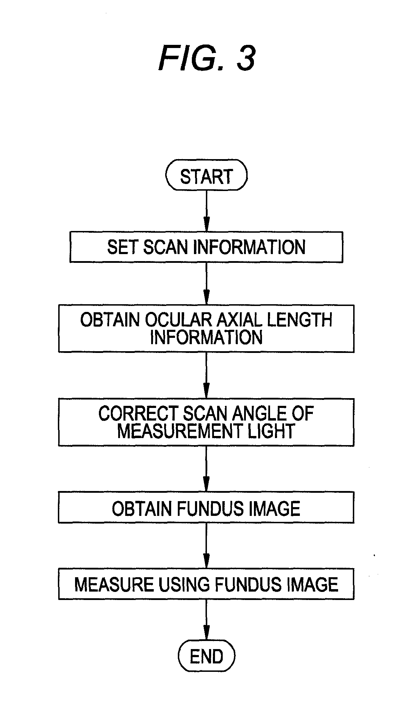

Fig. 3 is a flowchart showing an exemplary procedure for measuring a fundus image by changing operation of the optical scanner 102 in accordance with an ocular axial length. First, the scan information (e.g., pattern, angle, speed) of the measurement light that scans the fundus Ef is set. Subsequently, the control part 70 obtains length information (ocular axial length information) of the eye E, and then corrects the scan information of the measurement light based on the obtained length information. For example, the control part 70 corrects the scan angle such that the fundus image is obtained at a photographing range that is set. Since a change in the scan angle changes the photographing range on the fundus, the scan angle is corrected such that a shift of the photographing range due to the difference in eye length is corrected.

The control part 70 outputs a driving signal corresponding to the corrected scan information to the optical scanner 102. Then, the control part 70 operates the optical scanner 102 to obtain a desired fundus image. The fundus image may be obtained as a still image, or fundus images may be obtained continuously as moving images.

The scan information (e.g., pattern, angle, speed) of the measurement light and the driving signal (e.g., pattern, range, speed) to be output to the optical scanner 102 are associated in advance, and are stored in the memory 72. A specific example will be described in detail below.

### <Setting of scan information of measurement light>

In the case of obtaining the front image, for example, the photographing range t in each of vertical and horizontal directions is set. Then, an arbitrary region is two-dimensionally scanned with the measurement light (e.g., rectangular region of 8 mm × 8 mm is scanned in a raster manner with measurement light).

In the case of obtaining the tomographic image, for example, a scanning pattern and the photographing range t are set. The scanning pattern is selected from, for example, line scan (refer to L1 of Fig. 5), cross scan, radial scan, and circle scan. An arbitrary region may be two-dimensionally scanned with the measurement light (e.g., arbitrary rectangular region of 5 mm x 5 mm is scanned in a raster manner with measurement light).

As the photographing range t of the tomographic image, for example, a fundus scan length is set. For example, an arbitrary scan length is selected from a plurality of scan lengths (e.g., 3 mm, 6 mm, and 9 mm). Alternatively, an arbitrary scan length may be selected by an input of a numeric value corresponding to the scan length by an examiner. In the case of scanning in a line manner, the fundus scan length t, for example, is expressed by a distance from a scanning start position to a scanning end position on the fundus. In the case of scanning in a circular manner, the fundus scan length t, for example, is expressed by a diameter of the circle. In the case of scanning in a rectangular manner, the fundus scan length t, for example, is expressed by a scanning distance in a vertical direction and a scanning distance in a horizontal direction.

The foregoing scan information may be arbitrarily set by the examiner or may be set in advance. Moreover, a scaling factor having a certain photographing range as a reference may be set as the scan information.

### <Obtainment of ocular axial length X>

The ocular axial length X is measured by an ocular axial length measurement apparatus (e.g., optical interference apparatus, or ultrasonic wave apparatus). For example, in the case where the measurement apparatus 110 is provided independently from the present apparatus, the measurement apparatus 110 and the present apparatus may be connected through a communication line. In such a case, a value of the ocular axial length is input to the present apparatus by data transfer. The ocular axial length may be obtained by a manual input using an operating part. The ocular axial length may be obtained from a server storing a measurement value therein. As shown in Fig. 1, the measurement apparatus 110 can be included in the present apparatus. The ocular axial length may be measured in advance. For example, after changing an optical path length, the control part 70 may obtain the length information based on positional information of an optical path length variable optical member (e.g., position of a reference mirror 31 of Fig. 7) at the time of obtaining the interference signal corresponding to the fundus. The ocular axial length may be determined complimentarily based on a diopter scale, a corneal curvature, and a crystalline lens power of the eye E, and the like.

### <Calculation of correction amount of scan angle>

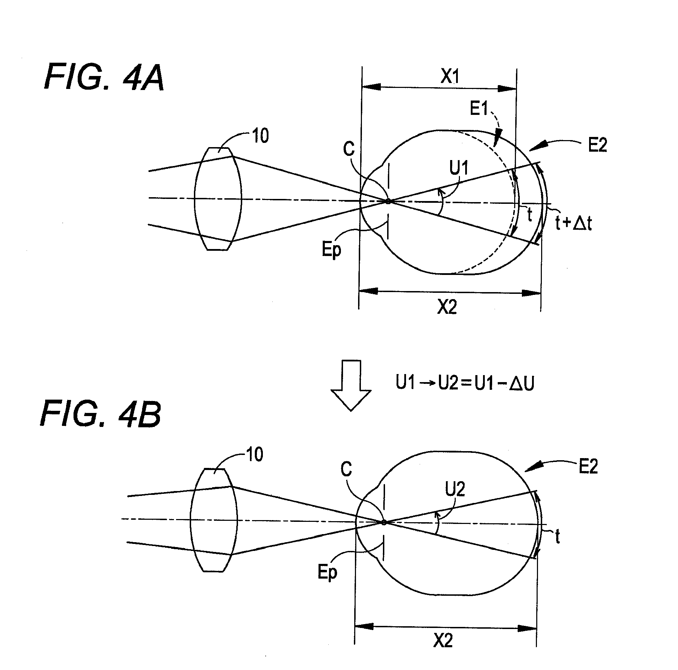

Figs. 4A and 4B are schematic explanatory diagrams showing an exemplary calculation technique to determine the scan angle corresponding to the ocular axial length X. The scan angle U with respect to the fundus E is calculated based on the set scan length t and the ocular axial length X.

As shown in Fig. 4A, in the case where an ocular axial length of an eye E1 is X1 (e.g., average ocular axial length of Japanese is 24 mm), a fundus image of the photographing range t is obtained by scanning a range corresponding to a scan angle U1 with light (refer to Fig. 5). The scan angle U1 and the scan length t, for example, are determined by a relationship between the scanning view angle and the photographing range in the optical system of the present apparatus by use of a calibration optical member (e.g., model eye) having the known ocular axial length X1. The scan angle U1 and the scan length t may be determined by simulation using light ray tracking technique.

In the case where an ocular axial length of an eye E2 is X2 (> X1), a range corresponding to the scanning view angle U1 is scanned with the measurement light, thereby photographing a fundus image having a photographing range with a scan length of t + Δt. The photographing range is greater than the set scan length t by an amount of Δt. Since the eye E2 has a greater intraocular distance than the eye E1, a position of the measurement light at the time of reaching the fundus is higher. This leads to the greater photographing range.

Figs. 6A and 6B are examples showing a change of the photographing range in accordance with the ocular axial length. Fig. 6A shows a front image, whereas Fig. 6B shows a tomographic image. Each of image regions G1 and G2 indicated by dotted lines corresponds to the photographing range in the case of the ocular axial length of X1. The photographing ranges (refer to solid lines) in the case of the ocular axial length of X2 are larger than the image regions G1 and G2.

An image region corresponding to a predetermined photographing range may be extracted from the fundus image by image processing. A scale factor of the extracted image region may be adjusted in accordance with size of an image as whole. Such an adjustment, however, consumes an extra time of the image processing and decreases the number of measurement points, causing the possibility of generating differences in accuracy of various measurements (e.g., thickness, length, and area).

Fig. 4B is a schematic diagram showing a scan angle after correction thereof is made. For subtraction of the increment Δt relative to the scan length t, a correction amount ΔU is subtracted from the scanning view angle U1. This allows the scanning view angle to be corrected to a scanning view angle U2 (U2 = U1 - ΔU). The correction amount ΔU is determined by a relational expression of Δt = qΔU (q is a coefficient that varies with the ocular axial length). The correction amount ΔU fluctuates with a shifted amount of the ocular axial length from X1. Such a calculation is performed based on a result of light ray tracking of the measurement light that scans the fundus. For example, a value determined by a model eye is used as optical data of an eye.

### <Obtainment of fundus image by use of corrected scan angle>

The control part 70 operates the optical scanner 102 to control a traveling direction of the measurement light. This control is performed such that the fundus Ef is scanned with the light at the scanning view angle U2 at a predetermined frame rate. The light including the reflected light from the fundus Ef is received by the light receiving device 104. The control part 70 obtains the fundus image based on the light receiving signal output from the light receiving device 104.

Accordingly, the scan information of the measurement light is corrected in accordance with the ocular axial length, so that an image corresponding to a desired photographing range is obtained. The correction of the scan angle and the obtainment of the image at substantially the same scanning speed/frame rate allow the image based on the same number of measurement points in accordance with the desired scan length to be obtained (refer to Fig. 5).

The description of the present embodiment has been made on the case where the fundus is scanned along one scanning direction. In the case of obtaining the front image, the control part 70 corrects the scan angle with respect to each of the vertical and horizontal scanning directions. In the case of obtaining the tomographic image, the control part 70 corrects the scan angle with respect to each scanning direction. In the case of obtaining the two-dimensional tomographic image, the control part 70 corrects the scan angle with respect to each of the vertical and horizontal scanning directions.

### <Measurement of actual distance>

The obtained fundus image is stored in the memory 72 and displayed on the display monitor 75. The control part 70 performs calculation process for measuring an actual distance between two points on a fundus by use of a fundus image that is arbitrarily selected from at least any of a tomographic image Gt and a front image Gf.

When the two arbitrary points on the image (refer to points A and B of Fig. 5) are designated by operation (e.g., click operation) of the mouse 74a and the like, the control part 70 converts a distance between the designated two points into an actual distance on the fundus. Alternatively, the control part 70 may convert a distance between two markers (indicators) that are movably displayed on the image into the actual distance on the fundus.

The scan length of the fundus image is constant, thereby maintaining a certain level of measurement accuracy in various measurements. Therefore, analysis/analytical study can be performed in a more quantitative manner.

A technique for designating two arbitrary points for the actual distance measurement may be variously changed and is not limited to the technique described above. For example, the control part 70 may use a circular marker so as to designate two arbitrary points. In such a case, the control part 70 needs to determine a radius or a diameter of the marker so as to determine the distance between the two points. The control part 70 may measure a shape or area formed by three or more points including the above two arbitrary points and another point in a depth direction. The control part 70 may perform measurement of XY directions on a three-dimensional image (e.g., measurement with respect to a layer thickness map). In addition, the control part 70 may specify a measured portion by detection of a given portion of a tomographic image by image processing.

The description of the present embodiment has been made on the case where the eye E has the ocular axial length that is longer than X1. However, in the case of photographing the eye E having an ocular axial length that is shorter than X1, the foregoing technique can be used. In the case of no correction, the scan length is expressed by t - Δt. ΔU corresponding to a decrement Δt from the scan length t is added to the scanning view angle U1. Therefore, the scanning view angle is corrected to a scanning view angle U3 (U3 = U1 + ΔU).

The control part 70 may determine the correction amount ΔU for the correction of the scanning view angle U corresponding to the ocular axial length X by use of a predetermined calculation expression or by use of a table associating the ocular axial length X with the scanning view angle U. Since the scanning view angle U is controlled by a driving signal of the optical scanner 102, a table associating the ocular axial length X with the driving signal of the optical scanner 102 may be used instead of the foregoing table. The ocular axial length information may not necessarily be an actual measurement value, as long as it is information associated with the ocular axial length. The ocular axial length information may be positional information of an optical path length variable member in an ocular axial length measurement apparatus. Moreover, the scan information may be corrected by software so as to correct the scan angle, or may be corrected by hardware such as a dedicated driving circuit (e.g., large scale integrated circuit: LSI).

The control part 70 may keep the scan angle constant while correcting the scanning speed of the measurement light at a predetermined frame rate in accordance with the ocular axial length. The control part 70 may keep the scanning speed constant while changing the frame rate at the time of obtaining the fundus image in accordance with the ocular axial length. The control part 70 may correct the photographing range by changing a lighting timing of the light source 101. These techniques lead to correction of a range to be photographed by optical scanning.

The control part 70 obtains at least any of the corneal shape information and the eye refractive power (diopter) information of the eye E, so that the scanning view angle may be corrected based on the obtained the corneal shape information/the diopter information. The smaller the corneal curvature radius (the greater the eye refractive power), the greater the refraction of the measurement light. This increases the photographing range. The control part 70, for example, corrects the scan angle in accordance with the corneal curvature radius and/or the eye refractive power such that the photographing range on the fundus becomes a predetermined photographing range. In the relational expression of Δt = qΔU, q is a coefficient that varies with the corneal shape/eye refractive power. As similar to the ocular axial length, the corneal shape/eye refractive power may be obtained by a member provided in the present apparatus. Alternatively, the corneal shape/eye refractive power may be obtained from another apparatus. The control part 70 may correct the scan information based on the eye length information and at least any of the diopter information and the corneal shape information.

Fig. 7 is a diagram showing a specific example (present specific example) of the optical system and the control system of the present apparatus. The photographing optical system 100 described above with reference to Fig. 1 includes an interference optical system (OCT optical system) 200 and a scanning laser ophthalmoscope (SLO) optical system 300 shown in Fig. 7. The OCT optical system 200 obtains a tomographic image by use of a light coherence tomography technique, whereas the SLO optical system 300 obtains a front image by use of infrared light. The photographing optical system 100 (OCT optical system 200 and SLO optical system 300) is arranged inside a housing (not shown). The housing is three-dimensionally moved with respect to the examinee's eye E by a known (manual or electrically-powered) movement mechanism for alignment.

It is to be noted that a dichroic mirror 40 is used as a light splitting member. The dichroic mirror 40 has a characteristic of reflecting measurement light (e.g., λ = about 840 nm) emitted from a measurement light source 27 provided in the OCT optical system 200, while being transmitted by laser light (light with a different wavelength from that of the light source 27, e.g., λ = about 780 nm) emitted from a light emitting part 61 provided in the SLO optical system 300. The dichroic mirror 40 makes a measurement optical axis L2 of the OCT optical system 200 and a measurement optical axis L1 of the SLO optical system 300 be the same axial.

A configuration of the OCT optical system 200 provided on the opposite side to the dichroic mirror 40 will be described. The OCT optical system 200 splits a light flux emitted from the light source into a measurement light flux and a reference light flux. Further, the OCT optical system 200 guides the measurement light flux to the fundus Ef, while guiding the reference light flux to the reference optical system. Subsequently, the OCT optical system 200 makes the light receiving device receive interference light obtained by combining the measurement light flux, reflected on the fundus Ef, with the reference light flux.

As the OCT optical system 200, there has been used an OCT optical system of a spectral domain type. Naturally, a time domain type (TD-OCT) or a swept source domain type (SS-OCT) may also be used.

The OCT light source 27 emits low coherent light. As the OCT light source 27, there is for example used a light source that emits light with a central wavelength of 840 nm and a band width of 50 nm (e.g., SLD light source). A fiber coupler 26 serves as a light splitting member as well as a light coupling member. The light emitted from the OCT light source 27 passes through an optical fiber 38a as a guiding optical path, and is thereafter split by the coupler 26 into reference light and measurement light. The measurement light travels toward the eye E via an optical fiber 38b, while the reference light travels toward a reference mirror 31 via an optical fiber 38c.

In an optical path for emitting the measurement light toward the eye E, an end 39b of the optical fiber 38b, a collimator lens 22, a focusing lens 24 and a scanning part 23 are arranged. The focusing lens 24 is movable in the optical-axis direction in line with a refraction error of the eye E for adjustment of a focus on the fundus. The scanning part 23 is capable of scanning the fundus in XY directions with the measurement light. This scanning part 23 includes two galvanometer mirrors, and is operated by driving of a scanning driving mechanism 51. The dichroic mirror 40 and an objective lens 10 serve as a light guiding optical system for guiding OCT measurement light from the OCT optical system 200 to the fundus. It is to be noted that the scanning part 23 of the present embodiment arbitrarily adjusts a reflection angle of the measurement light by means of the two galvanometer mirrors. Hence a direction of scanning by means of the measurement light on the fundus is arbitrarily set. A tomographic image in an arbitrary area of the fundus is thus obtained. It is to be noted that the end 39b of the optical fiber 38b is arranged in a position conjugate with the fundus of the eye E. Further, the two galvanometer mirrors of the scanning part 23 are arranged in a position substantially conjugate with a pupil of the eye E.

The galvanometer mirrors and the scanning driving mechanism 51 described above are used as an optical scanner (optical scanning part). The optical scanner is arranged in the optical path for the measurement light flux (measurement optical path). The optical scanner changes a traveling direction of the measurement light flux in order to scan the predetermined region of the eye in a transverse direction (XY directions) with the measurement light flux. As the optical scanner, other than the mirror, an acousto-optic device (AOM: Acousto-Optic Modulator) for changing a traveling (deflection) direction of light, and the like are used.

The measurement light emitted from the end 39b of the optical fiber 38b is collimated by the collimator lens 22, and thereafter reaches the scanning part 23 via the focusing lens 24. In this scanning part 23, the two galvanometer mirrors are driven, to change a reflecting direction of the measurement light. The measurement light reflected on the scanning part 23 is reflected on the dichroic mirror 40, and thereafter collected in the fundus via the objective lens 10.

The measurement light reflected on the fundus passes through the objective lens 10, and is thereafter reflected on the dichroic mirror 40, to travel toward the OCT optical system 200. Further, the measurement light is incident on the end 39b of the optical fiber 38b via the two galvanometer mirrors of the scanning part 23, the focusing lens 24 and the collimator lens 22. The measurement light incident on the end 39b reaches an end 84a of an optical fiber 38d via the optical fiber 38b, the fiber coupler 26 and the optical fiber 38d.

Meanwhile, in an optical path for emitting reference light toward the reference mirror 31 (reference optical path), an end 39c of the optical fiber 38c, a collimator lens 29 and the reference mirror 31 are arranged. The reference mirror 31 is configured to be movable in the optical-axis direction by a reference mirror driving mechanism 50. This allows the reference mirror 31 to change an optical path length of the reference light.

The reference light emitted from the end 39c of the optical fiber 38c is made to be a parallel light flux by the collimator lens 29 and reflected on the reference mirror 31, and is thereafter collected by the collimator lens 29, to be incident on the end 39c of the optical fiber 38c. The reference light incident on the end 39c reaches the coupler 26 via the optical fiber 38c.

The reference light generated as described above and the fundus reflected light obtained by reflection of the measurement light on the fundus are combined in the coupler 26, to become interference light. The interference light is emitted from the end 84a through the optical fiber 38d.

A spectroscopic optical system 800 (spectrometer part) splits the interference light into each frequency component for obtaining an interference signal with reference to each frequency. The spectroscopic optical system 800 has a collimator lens 80, a grating (diffraction grating) 81, a condenser lens 82 and a light receiving device (detector) 83. The light receiving device 83 includes a one-dimensional device (line sensor) having the sensitivity to light with a wavelength in an infrared region.

The light emitted from the end 84a is made to be parallel light in the collimator lens 80, and thereafter split in the grating 81 into each frequency component (each wavelength component). The split light is then collected on the light receiving surface of the light receiving device 83 via the condenser lens 82. Thereby, spectrum information with interference fringes is recorded in the light receiving device 83. The spectrum information (light receiving signal) is then input into a control part 70. The control part 70 can analyze the spectrum information by use of Fourier transformation, to measure information (A-scan signal) in the depth direction of the eye. Using the scanning part 23, the control part 70 can scan the fundus in a predetermined transverse direction with the measurement light, to obtain a tomographic image. For example, the control part 70 can scan the fundus in the X-direction or the Y-direction with the measurement light, to obtain a tomographic image in an X-Z plane or a Y-Z plane (it is to be noted that in the present embodiment, such a method for one-dimensionally scanning the fundus with the measurement light to obtain a tomographic image is referred to as B-scan). In addition, the obtained tomographic image is stored in a memory 72 connected to the control part 70. Further, the control part 70 can two-dimensionally scan the fundus in the XY directions with the measurement light, to obtain a three-dimensional image of the fundus. It is to be noted that, in the present embodiment, the OCT image is obtained by the two galvanometer mirrors provided in the scanning part 23.

Next, the SLO optical system (confocal optical system) 300 arranged in a transmitting direction of the dichroic mirror 40 will be described. The SLO optical system 300 is broadly divided into an illuminating optical system for illuminating the fundus and a light receiving optical system for receiving, with the light receiving device, reflected light from the fundus illuminated by the illuminating optical system. The SLO optical system 300 obtains a front image of the fundus based on a light receiving signal output from the light receiving device.

The light emitting part 61 has a first light source (SLO light source) 61a, a second light source (fixation optical system) 6 1 b, a mirror 69, and a dichroic mirror 101. The first light source 61a emits light with a wavelength in the infrared region (e.g., λ = 780 nm), and the second light source 61b emits light with a wavelength in a visible region (e.g., λ = 630 nm). It is to be noted that as the first light source 61a and the second light source 61 b, a light source is used which emits light with high luminance and high directivity (such as a laser diode light source or an SLD light source). Infrared light emitted from the first light source 61 a passes through the dichroic mirror 101, and travels to a beam splitter 62 through a collimator lens 65. Visible light emitted from the second light source 61b is bent by the mirror 69, and thereafter reflected on the dichroic mirror 101. This visible light then travels along the same axis as that of the infrared light emitted from the first light source 61 a. The first light source 61 a is used for obtaining a fundus front image for observation. Meanwhile, the second light source 61b is used for guiding the sight direction of the eye.

In the optical path for emitting laser light from the light emitting part 61 toward the eye E, the collimator lens 65, a focusing lens 63, the scanning part (optical scanner) 64 and the objective lens 10 are arranged. The focusing lens 63 is movable in the optical-axis direction in line with a refraction error of the eye. The scanning part 64 can perform high-speed scanning on the fundus in the XY directions with the measurement light. The scanning part 64 has a galvanometer mirror and a polygon mirror, and is driven by a scanning driving mechanism 52. Reflected surfaces of the galvanometer mirror and the polygon mirror can be arranged in a position substantially conjugate with the pupil of the eye E.

Further, the beam splitter 62 is arranged between the light emitting part 61 and the focusing lens 63. Moreover, on the reflecting direction of the beam splitter 62, a condenser lens 66, a confocal opening 67 and a light receiving device 68 for SLO are provided. The condenser lens 66 serves to configure the confocal optical system. The confocal opening 67 is arranged in a position conjugate with the fundus.

Herein, laser light (measurement light or fixation light) emitted from the light emitting part 61 transmits the beam splitter 62 via the collimator lens 65, and thereafter passes through the focusing lens 63. Subsequently, this laser light reaches the scanning part 64. By driving of the galvanometer mirror and the polygon mirror, the reflecting direction of this laser light is changed. The reflected laser light transmits the dichroic mirror 40, and is thereafter collected in the fundus via the objective lens 10.

The laser light (measurement light) reflected on the fundus passes through the objective lens 10, the galvanometer mirror and the polygon mirror of the scanning part 64 and the focusing lens 63, and is then reflected on the beam splitter 62. Subsequently, this laser light is collected in the condenser lens 66, and thereafter detected by the light receiving device 68 via the confocal opening 67. A light receiving signal generated in the light receiving device 68 is input into the control part 70. The control part 70 obtains the front image of the fundus based on the light receiving signal obtained in the light receiving device 68. The obtained front image is stored in the memory 72. It is to be noted that at the time of obtaining the front image (SLO image), scanning (sub-scanning) of laser light in a longitudinal direction by means of the galvanometer mirror provided in the scanning part 64 and scanning (main scanning) of laser light in a transverse direction by means of the polygon mirror are implemented.

In the present embodiment, the movement of the focusing lens in the optical-axis direction adjusts the focus. However, a mechanism for adjusting the focus is not limited thereto, and may be a focusing optical member capable of adjusting an image forming state of an optical system. For example, a mirror unit that deflects received light flux by use of two mirrors may be configured to be moved in an optical-axis direction. Alternatively, not only may a plurality of lens having different diopter scales be removably arranged, but also a lens corresponding to the eye E may be arranged in an optical path.

It is to be noted that the control part 70 is connected to the display monitor 75, and controls a display image thereof. Further, the control part 70 is connected with a memory (storing part) 72, an operating part 74 for performing a variety of operations, the scanning driving mechanism 51, the scanning driving mechanism 52, the reference mirror driving mechanism 50, a first driving mechanism 63a for moving the focusing lens 63 in the optical-axis direction, a second driving mechanism 24a for moving the focusing lens 24 in the optical-axis direction, and the like. It is to be noted that as the monitor 75, two monitors, i.e., a monitor for alignment observation and a monitor for photographed image observation, may be used or one shared monitor may naturally be used. It is to be noted that the measurement position setting part (e.g., mouse) 74a and the photographing start switch 74b are provided in the operating part 74.

The control part 70 performs image processing on the light receiving signal output from the light receiving device 83 to form a fundus tomographic image Gt. Further, the control part 70 performs image processing on the light receiving signal output from the light receiving device 68 to form a fundus front image Gf (refer to Fig. 5).

A description is now given of operation of the apparatus having the above configuration. The control part 70 controls driving of the OCT optical system 200 and the SLO optical system 300. This allows the control part 70 to obtain an OCT image and an SLO image per frame. The control part 70 controls the display of the display monitor 75 to update the OCT image and the SLO image to be displayed on the display monitor 75 as the need arises (refer to Fig. 5).

The fundus image is obtained based on the scan information that is set in advance. Before the fundus image is obtained, operation for determining a various settings may be performed. Alternatively, the image may be obtained by a default setting. The ocular axial length of the eye E may be measured in advance by the measurement apparatus 110. In the case where the eye E has the known ocular axial length, the control part 70 corrects scan angle data relating to the OCT optical system 200. The control part 70 controls the scanning driving mechanism 51 based on the corrected scan angle data. In the present specific example, the control part 70 does not correct the scan angle of the SLO optical system 300.

The examiner instructs the examinee to gaze at the fixation light, and then performs alignment operation using a joystick (not shown), so that a measurement optical axis L1 is arranged in a pupil center of the eye to be examined. The examiner performs the alignment operation while observing an anterior-segment, displayed on the display monitor 75, to be photographed by an anterior-segment observing camera (not shown). Accordingly, the alignment of the measurement optical axis L1 with respect to the eye to be examined is complete. Then, the SLO optical system 300 obtains the front image of the fundus (SLO fundus image) of the eye to be examined. The SLO fundus image is displayed on the display monitor 75.

The control part 70 controls driving of the driving mechanism 50 based on the light receiving signal output from the light receiving device 83, thereby adjusting a difference between the optical path of the measurement light and the optical path of the reference light. This allows the fundus tomographic image to be obtained from a desired photographing position. The control part 70 allows the reference mirror 31 to be moved within a predetermined movement range corresponding to the difference in ocular axial length of the eye to be examined. It is to be noted that the reference mirror 31 may be moved to a position corresponding to the ocular axial length data obtained by the measurement apparatus 110. The control part 70 can also calculate the ocular axial length based on the position of the reference mirror 31 at the time of obtaining the tomographic image. In such a case, the control part 70 corrects the scan angle based on the calculated ocular axial length.

When the tomographic image is displayed on the display monitor 75, the examiner operates the measurement position setting part 74a while looking at the front image on the display monitor 75. This allows the examiner to set the scan information (e.g., position, range, scanning pattern, and scan length) of the measurement light (refer to line L1 of Fig. 5). The control part 70 allows the line L1 serving as information of a measurement position of the tomographic image to be superimposingly displayed on the front image.

In the present specific example, the scan angle of the SLO optical system 300 is not corrected. In the case of the constant scan angle, the photographing range of the front image varies with the ocular axial length. At the time of setting the photographing range of the tomographic image, therefore, the control part 70 corrects a display range of the line L1 (e.g., from scanning start point to scanning end point) on the fundus front image in accordance with the ocular axial length such that the display range of the line L1 corresponds to a measurement position of the OCT optical system 200.

When the scan information is set, the control part 70 corrects the scan angle based on the set scan information and the ocular axial length of the eye E. The control part 70 controls the scanning driving mechanism 51 based on the corrected scan angle, so that the tomographic image corresponding to the desired photographing region is obtained from the desired photographing position. Upon output of a trigger signal from the photographing start switch 74b, the control part 70 stores the tomographic image and the front image as still images in the memory 72. The control part 70 may generate an averaged image by obtaining a plurality of tomographic images.

In the present specific example, the control part 70 may allow the photographing range (e.g., scan length) to be displayed with a numerical value. In an early stage, the control part 70 may fix a length of the information on the measurement position of the tomographic image (e.g., length of line L1) to be displayed on the front image regardless of the ocular axial length. In a later stage, the control part 70 may change the numeric value indicating the photographing range in accordance with the ocular axial length. Alternatively, the control part 70, in the early stage, may fix the photographing range and change the length of the information on the measurement position of the tomographic image (e.g., length of line L1) to be displayed on the front image in accordance with the ocular axial length.

The description of the present specific example has been made on the example where the line L1 corresponding to the line scan is displayed. By using the foregoing display control, the control part 70 may also allow the information on the measurement position of the tomographic image corresponding to other scan patterns such as radial scan, circle scan, and two-dimensional rectangular scan to be displayed on the front image.

In the present specific example, in the case of displaying the front image, the control part 70 changes a display scaling factor of the front image in accordance with the ocular axial length, so that an image region corresponding to a certain photographing range can also be displayed on a front image display region on the display monitor 75.

In the present specific example, the scan angle of the SLO optical system 300 is not corrected in accordance with the ocular axial length. The SLO optical system 300 can obtain the front image with an optical scanner, such as a polygon mirror and a resonant scanner, capable of scanning at high speed. Therefore, the SLO optical system 300 not only can obtain a good front image at a high speed, but also can correct the photographing range of the tomographic image in accordance with the ocular axial length.

In the present specific example, the scan angle of the SLO optical system 300 is not corrected. However, the scan angle of the SLO optical system 300 can be corrected. As an optical scanner of the SLO optical system 300, for example, a galvanometer mirror capable of readily changing a scan angle is used.

In the present specific example, the SLO optical system 300 is used as a configuration to obtain the front image. Alternatively, a fundus camera-type configuration that irradiates an entire fundus with infrared light to obtain a fundus image by use of a photographing device may be used. Moreover, the technique of the present apparatus can also be applied to an apparatus that forms a fundus front image for observation based on a light receiving signal obtained by the OCT optical system 200.

In the present embodiment, the movement of the reference mirror 31 serving as the optical path length variable optical member changes the optical path length of the reference light, thereby adjusting the difference between the optical path length of the reference light and the optical path length of the measurement light. However, the adjustment is not limited thereto. The difference between the optical path length of the measurement light and the optical path length of the reference light may be changed by an optical path length changing member arranged in any of the reference optical path and the measurement optical path.

The description of the present embodiment has been made on the case where the fundus image is photographed, but is not limited thereto. The present apparatus may be used to photograph in the case where a distance between a scanning-type photographing optical system and an object to be examined has the possibility to change. For example, positional information of the object to be examined is obtained, and then scan information (e.g., scan angle) of measurement light is corrected based on the obtained positional information. The positional information of the object to be examined is, for example, obtained from positional information of an optical path length variable optical member. Alternatively, the positional information of the object to be examined may be obtained from an output signal from a sensor capable of measuring a distance between the object to be examined and the apparatus, or obtained from manual inputs by an examiner.

As described above, upon correction of the scan information of the measurement light in accordance with the distance to the object to be examined, the image corresponding to the desired photographing range is obtained. It is to be noted that an object to be measured (photographed) is considered to be, for example, a living body such as an anterior segment, skin, and an interior organ, and a sample other than the living body.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

## Claims

1. A fundus photographing apparatus for photographing a fundus of an examinee's eye, comprising:
a fundus photographing optical system (100) for obtaining a fundus image, including:
an optical scanner (102) that scans the fundus with measurement light including at least part of light emitted from a light source (101); and
a light detector (104) that receives light including reflected light from the fundus;
a length information obtaining unit (110) for obtaining length information on an axial direction of the eye; and
a controller that adjusts driving information of the fundus photographing optical system in relation to a photographing range based on the length information obtained by the length information obtaining unit and controls the fundus photographing optical system based on the adjusted driving information to obtain a fundus image corresponding to a photographing range.

2. The fundus photographing apparatus according to claim 1, wherein the controller is connected to the optical scanner, corrects scan information of the optical scanner based on the length information and controls the optical scanner based on the corrected scan information.

3. The fundus photographing apparatus according to claim 2, wherein the controller changes a scan angle of the optical scanner based on the length information.

4. The fundus photographing apparatus according to claim 1, further comprising:
a setting unit (74a), connected to the controller, for arbitrarily setting the photographing range of the fundus,
wherein the controller adjusts the driving information based on the photographing range set by the setting unit and the length information obtained by the length information obtaining unit to obtain a fundus image corresponding to the set photographing range.

5. The fundus photographing apparatus according to claim 1, wherein the fundus photographing optical system is an optical coherence tomography optical system (200) for obtaining a fundus tomographic image, the optical coherence tomography optical system including:
a splitter (26) that splits the light emitted from the light source into light of a measurement optical path and light of a reference optical path; and
a light detector (83) that receives light obtained by combine of the light from the measurement optical path and the light from the reference optical path, the light from the measurement optical path being reflected from the fundus.

6. The fundus photographing apparatus according to claim 5, further comprising:
a front image photographing optical system (300) that photographs a front image of the fundus; and
a setting unit (74a), connected to the controller, for setting information on a measurement position of a tomographic image to be displayed on the fundus image displayed on a monitor,
wherein the controller adjusts the driving information based on the measurement position information set by the setting unit and the length information obtained by the length information obtaining unit to obtain a fundus image corresponding to a photographing range corresponding to the set measurement position information.

7. The fundus photographing apparatus according to claim 6, wherein the controller allows a pattern indicting the measurement position information set by the setting unit to be displayed on the fundus image, and changes a display region of the pattern on the fundus front image based on the length information obtained by the length information obtaining unit.

8. The fundus photographing apparatus according to claim 5, wherein the length information obtaining unit obtains the length information based on positional information of an optical member that is arranged to change a difference in optical path length between the reference optical path and the measurement optical path.

9. The fundus photographing apparatus according to claim 1, further comprising:
an eye information obtaining unit (110) that obtains at least any of diopter information and corneal shape information of the eye,
wherein the controller adjusts the driving information based on the length information obtained by the length information obtaining unit and at least any of the diopter information and the corneal shape information obtained by the eye information obtaining unit.
